(19) Europäisches Patentamt
European Patent Office
Office européen des brevets

(11) Veröffentlichungsnummer: **0 023 286**
**B1**

(12) # EUROPÄISCHE PATENTSCHRIFT

(45) Veröffentlichungstag der Patentschrift: **13.04.83**

(21) Anmeldenummer: **80103844.9**

(22) Anmeldetag: **07.07.80**

(51) Int. Cl.³: **C 07 D 249/08,** C 07 D 233/60, A 01 N 43/50, A 01 N 43/64 // C07C49/255, C07C49/175

(54) **Azolyl-alkenole, Verfahren zu ihrer Herstellung sowie ihre Verwendung als Fungizide.**

(30) Priorität: **18.07.79 DE 2928967**

(43) Veröffentlichungstag der Anmeldung: **04.02.81 Patentblatt 81/5**

(45) Bekanntmachung des Hinweises auf die Patenterteilung: **13.04.83 Patentblatt 83/15**

(84) Benannte Vertragsstaaten: **AT BE CH DE FR GB IT LI NL**

(56) Entgegenhaltungen:
**EP-A-0 010 674**
**EP-A-0 022 969**
**GB-A-2 004 276**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Jäger, Gerhard, Dr., Gellertstrasse 18, D-5090 Leverkusen 1 (DE)**
Erfinder: **Kraatz, Udo, Dr., Körner Strasse 6, D-5090 Leverkusen 1 (DE)**
Erfinder: **Büchel, Karl Heinz, Prof.Dr., Haus an der Dachsdelle Dabringhauser Strasse 42, D-5093 Burscheid (DE)**
Erfinder: **Frohberger, Paul-Ernst, Dr., Willi-Baumeisterstrasse 6, D-5090 Leverkusen 1 (DE)**
Erfinder: **Brandes, Wilhelm, Dr., Eichendorffstrasse 3, D-5623 Leichlingen (DE)**
Erfinder: **Paul, Volker, Dr., Ahornstrasse 5, D-5650 Solingen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 023 286 B1

BUNDESDRUCKEREI BERLIN

Azolyl-alkenole, Verfahren zu ihrer Herstellung sowie ihre Verwendung als Fungizide

Die vorliegende Erfindung betrifft neue Azolyl-alkenole, ein Verfahren zu ihrer Herstellung sowie ihre Verwendung als Fungizide.

Es ist bereits bekanntgeworden, daß Trityl-triazole, wie Triphenyl-(1,2,4-triazol-1-yl)-methan, eine gute fungizide Wirksamkeit besitzen (vgl. DE-OS 1 795 249). Außerdem ist bereits bekanntgeworden, daß 1-Propyl-triazolyl- bzw. -imidazolyl-Derivate, wie insbesondere im Phenylteil substituierte 1-(Imidazol-1-yl)- bzw. (1,2,4-Triazol-1-yl)-2-phenoxy-4,4-dimethyl-pentan-3-ole, gute fungizide Eigenschaften aufweisen (vgl. DE-OS 2 350 122 bzw. 2 350 123). Die Wirkung dieser Verbindungen ist jedoch, insbesondere bei niedrigen Aufwandmengen und -konzentrationen, nicht immer ganz befriedigend.

Es wurden neue Azolyl-alkenole der Formel

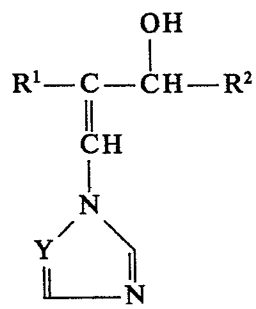

(I)

in welcher

$R^1$ für Phenoxy oder Phenyl steht, wobei die genannten Reste substituiert sein können durch Halogen, Alkyl mit 1 bis 4 Kohlenstoffatomen und durch gegebenenfalls halogensubstituiertes Phenyl,

$R^2$ für Alkyl mit 1 bis 4 Kohlenstoffatomen oder für Phenyl steht, wobei letzteres substituiert sein kann durch Halogen, Alkyl mit 1 bis 4 Kohlenstoffatomen und durch gegebenenfalls halogensubstituiertes Phenyl, und

Y für ein Stickstoffatom oder die CH-Gruppe steht,

und deren physiologisch verträglichen Säureadditions-Salze und Metallsalz-Komplexe gefunden.

Die Verbindungen der Formel (I) können in zwei geometrischen Isomerenformen vorliegen, je nach Anordnung der Gruppen, welche an die Kohlenstoffatome gebunden sind, die durch die Doppelbindung verbunden sind. Die Verbindungen der Formel (I) besitzen ein asymmetrisches Kohlenstoffatom und können deshalb auch in den beiden optischen Isomeren oder als Racemate vorliegen. Sämtliche Isomeren sowie deren Gemische werden erfindungsgemäß beansprucht.

Weiterhin wurde gefunden, daß man die Azolyl-alkenole der Formel (I) erhält, wenn man Azolyl-alkenone der Formel

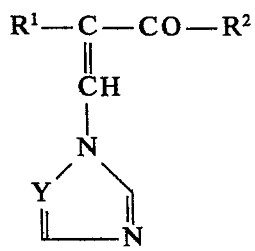

(II)

in welcher

$R^1$, $R^2$ und Y die oben angegebene Bedeutung haben,

nach bekannten Methoden in üblicher Weise reduziert. An die so erhaltenen Verbindungen der Formel (I) kann gegebenenfalls anschließend eine Säure oder ein Metallsalz addiert werden.

Die neuen Azolyl-alkenole der Formel (I) weisen gute fungizide Eigenschaften auf. Überraschenderweise zeigen die erfindungsgemäßen Verbindungen eine erheblich höhere fungizide Wirksamkeit als die aus dem Stand der Technik bekannten Verbindungen Triphenyl-(1,2,4-triazol-1-yl)-methan und im Phenylteil substituierte 1-(Imidazol-1-yl)- bzw. -(1,2,4-Triazol-1-yl)-2-phenoxy-4,4-dimethyl-pentan-3-ole, welche chemisch und wirkungsmäßig naheliegende Verbindungen sind. Die erfindungsgemäßen Stoffe stellen somit eine Bereicherung der Technik dar.

Antimykotische Mittel, enthaltend die obengenannten Azolyl-alkenole, sind in der parallelen EP-A-0 022 969 gleicher Priorität beansprucht.

Die erfindungsgemäßen Azolyl-alkenole sind durch die Formel (I) allgemein definiert. Bevorzugt

sind diejenigen Verbindungen der Formel (I), in denen $R^1$ für jeweils gegebenenfalls einfach oder mehrfach, gleich oder verschieden durch Fluor, Chlor, Brom, Methyl, Ethyl, Phenyl oder Chlorphenyl substituiertes Phenyl oder Phenoxy steht, $R^2$ für Methyl, Isopropyl, tert.-Butyl sowie für gegebenenfalls einfach oder mehrfach, gleich oder verschieden durch Fluor, Chlor, Brom, Methyl, Ethyl oder Phenyl substituiertes Phenyl steht, und Y für ein Stickstoffatom oder die CH-Gruppe steht.

Im einzelnen seien außer den bei den Herstellungsbeispielen genannten Verbindungen die folgenden Verbindungen der allgemeinen Formel (I) genannt:

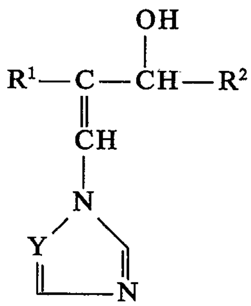

(I)

| $R^1$ | $R^2$ | Y |
|---|---|---|
| 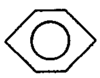 | 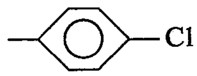 | N |
| 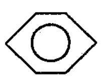 | 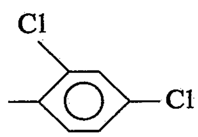 | N oder CH |
| 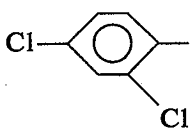 | 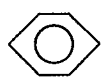 | N oder CH |
| 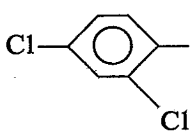 | 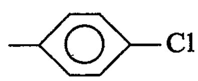 | N oder CH |
| 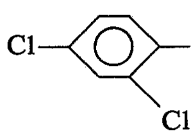 | 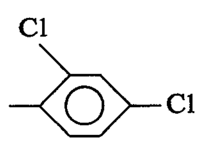 | N oder CH |
| 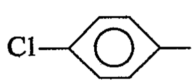 | 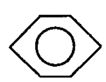 | N oder CH |
| 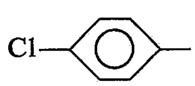 | 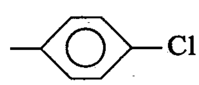 | N oder CH |
| 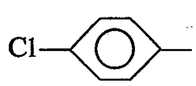 | 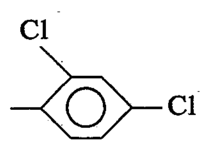 | N oder CH |
| 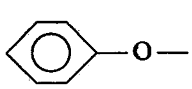 | $C(CH_3)_3$ | N |

Fortsetzung

| $R^1$ | $R^2$ | Y |
|---|---|---|
| 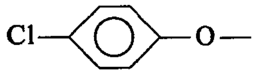 | $C(CH_3)_3$ | N |
| 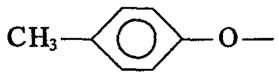 | $C(CH_3)_3$ | N |
| 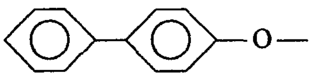 | $C(CH_3)_3$ | N |
| 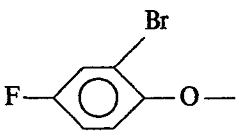 | $C(CH_3)_3$ | N |
| 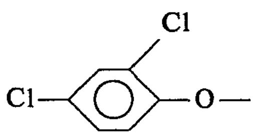 | $C(CH_3)_3$ | N |
| 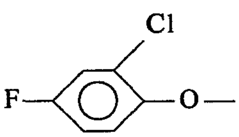 | $C(CH_3)_3$ | N |
| 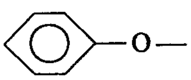 | 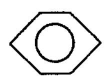 | N oder CH |
| 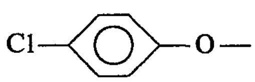 | 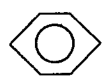 | N oder CH |
| 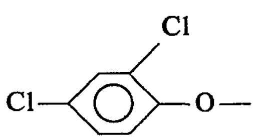 | 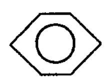 | N oder CH |
| 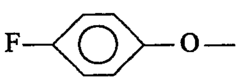 | 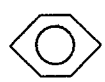 | N oder CH |
| 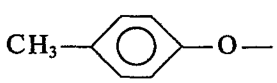 | 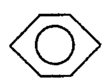 | N oder CH |
| 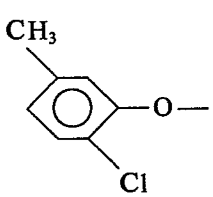 | 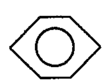 | N oder CH |
| 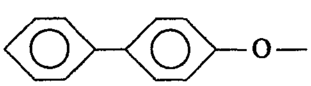 | 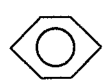 | N oder CH |

Verwendet man beispielsweise 1-(Imidazol-1-yl)-2-(4-fluorphenoxy)-4,4-dimethyl-1-penten-3-on und Natriumborhydrid als Ausgangsstoffe, so kann der Reaktionsablauf durch das folgende Formelschema wiedergegeben werden:

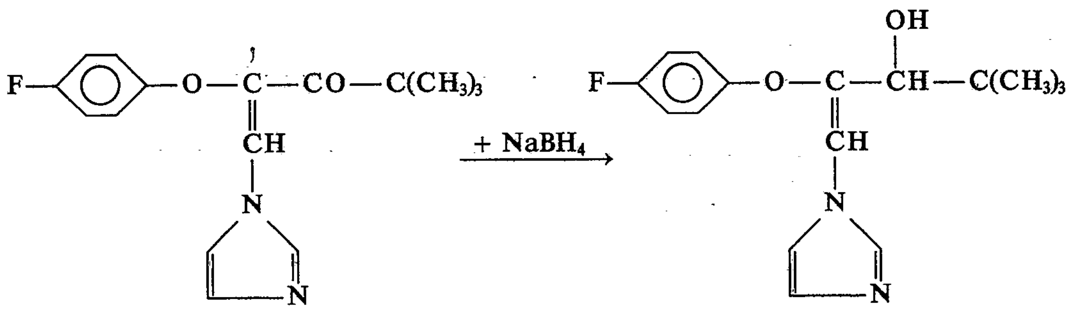


Die für das erfindungsgemäße Verfahren als Ausgangsstoffe zu verwendenden Azolyl-alkenone sind durch die Formel (II) allgemein definiert. In dieser Formel stehen $R^1$, $R^2$ und Y vorzugsweise für die Reste, die bei den Verbindungen der Formel (I) bereits vorzugsweise für diese Substituenten genannt wurden.

Die Azolyl-alkenone der Formel (II) sind Gegenstand einer eigenen älteren Anmeldung (veröffentlichte Europäische Patentanmeldung Nr. 0 010 674). Die Verbindungen werden erhalten, indem man 1-Halogen-ethen-Derivate der Formel

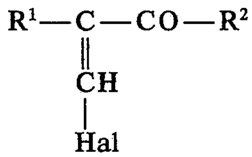
(III)

in welcher

$R^1$ und $R^2$ die oben angegebene Bedeutung haben und
Hal für Halogen, vorzugsweise Chlor oder Brom, steht,

mit Alkalisalzen von Azolen der Formel

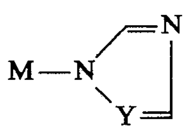
(IV)

in welcher

Y die oben angegebene Bedeutung hat und
M für ein Alkalimetall, vorzugsweise Natrium oder Kalium, steht,

in Gegenwart eines inerten organischen Lösungsmittels, wie beispielsweise Acetonitril, bei Temperaturen zwischen 20 und 120°C umsetzt. Die Isolierung der Verbindungen der Formel (II) erfolgt in üblicher Weise, wobei eine Reinigung gegebenenfalls über ein Säureadditionssalz durchgeführt werden kann (vgl. auch die Herstellungsbeispiele).

Die Azolyl-alkenone der Formel (II) können auch nach einem eigenen neuen Verfahren erhalten werden, indem man 1-Halogen-ethen-Derivate der Formel

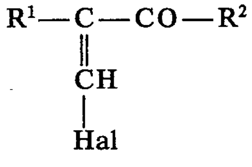
(III)

in welcher
$R^1$, $R^2$ und Hal die oben angegebene Bedeutung haben,
mit Trimethylsilyl-azolen der Formel

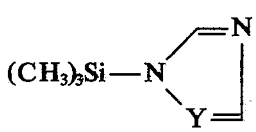
(V)

in welcher
Y die oben angegebene Bedeutung hat,
in Gegenwart eines Verdünnungsmittels umsetzt.

Die 1-Halogen-ethen-Derivate der Formel (III) sind teilweise bekannt und können in allgemein bekannter Art und Weise erhalten werden, wenn man entsprechende Ethen-Derivate der Formel

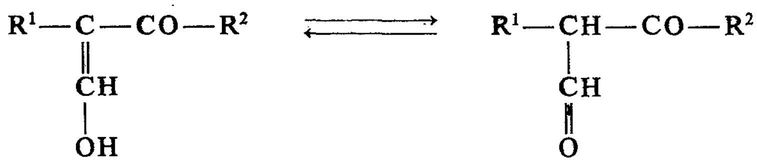
(VI)

in welcher
$R^1$ und $R^2$ die oben angegebene Bedeutung haben,
mit einem Halogenierungsmittel, wie Phosphor- und Schwefelhalogeniden, beispielsweise seien Thionylchlorid, Sulfurylchlorid, Phosphortrichlorid oder -bromid und Phosphoroxychlorid genannt, gegebenenfalls in Gegenwart eines Verdünnungsmittels, wie beispielsweise Toluol oder Xylol, bei Temperaturen zwischen 20 und 100°C umsetzt (vgl. auch die Herstellungsbeispiele).

Die 1-Hydroxy-ethen-Derivate der Formel (VI) sind teilweise bekannt (vgl. u. a. Liebigs Ann. Chem., 379, 230 [1911]) bzw. können in allgemein bekannter Art und Weise erhalten werden, indem man bekannte Ketone der Formel

$$R^1-CH_2-CO-R^2 \qquad (VII)$$

in welcher
$R^1$ und $R^2$ die oben angegebene Bedeutung haben,
mit Ameisensäureestern der Formel

$$H-CO-OR^3 \qquad (VIII)$$

in welcher
$R^3$ für Methyl oder Ethyl steht,
in Gegenwart von Natriummethylat bzw. -ethylat in Methanol bzw. Ethanol bei Temperaturen zwischen 0 und 40°C umsetzt (vgl. auch die Herstellungsbeispiele).

Die Alkalisalze von Azolen der Formel (IV) sind bekannt. Sie werden durch Umsetzung von Imidazol bzw. 1,2,4-Triazol mit Natrium- oder Kaliummethylat in Methanol oder durch Umsetzung von Imidazol mit der äquivalenten Menge des entsprechenden Alkalihydrids erhalten.

Die Trimethylsilyl-azole der Formel (V) sind ebenfalls bekannt (vgl. DE-OS 1 940 628). Sie werden durch Umsetzung von Imidazol bzw. 1,2,4-Triazol mit Trimethylchlorsilan in Gegenwart einer Base erhalten (vgl. auch Chem. Ber., 93, 2804).

Als Verdünnungsmittel kommen für die neue Umsetzung der 1-Halogen-ethen-Derivate der Formel (III) mit den Trimethylsilyl-azolen der Formel (V) vorzugsweise inerte, organische Lösungsmittel in Frage. Hierzu gehören vorzugsweise aromatische Kohlenwasserstoffe, wie Benzol oder Toluol; Nitrile, wie Acetonitril; Ketone, wie Aceton; Ether, wie Diethylether; sowie Dimethylformamid.

Die Reaktionstemperaturen können bei dieser Umsetzung in einem größeren Bereich variiert werden. Im allgemeinen arbeitet man zwischen etwa 10 bis 120°C, vorzugsweise bei der Siedetemperatur des verwendeten Lösungsmittels.

Bei der Durchführung dieser Umsetzung arbeitet man vorzugsweise in molaren Mengen. Die Isolierung der Verbindungen der Formel (II) erfolgt in üblicher Weise.

Die erfindungsgemäße Reduktion erfolgt in üblicher Weise, wie z. B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30°C, vorzugsweise bei 0 bis 20°C, durchgeführt. Hierzu setzt man auf 1 Mol des Ketons der Formel (I) etwa 1 Mol eines komplexen Hydrids, wie Natriumhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (I) etwa 1 bis 2 Mol Aluminiumisopropylat ein. Zur

Isolierung der reduzierten Verbindungen der Formel (I) wird das überschüssige Lösungsmittel durch Destillation im Vakuum entfernt und die entstandene Aluminium-Verbindung mit verdünnter Schwefelsäure oder Natronlauge zersetzt. Die weitere Aufarbeitung erfolgt in üblicher Weise.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronsäure, Salizylsäure, Sorbinsäure, Milchsäure, sowie Sulfonsäuren, wie z. B. p-Toluolsulfonsäure und 1,5-Naphthalindisulfonsäure.

Die Salze der Verbindungen der Formel (I) können in einfacher Weise nach üblichen Salzbildungsmethoden, z. B. durch Lösen einer Verbindung der Formel (I), in einem geeigneten inerten Lösungsmittel und Hinzufügen der Säure, z. B. Chlorwasserstoffsäure, erhalten und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich von physiologisch verträglichen Säuren ableiten. Hierzu gehören vorzugsweise die Halogenwasserstoffsäuren, wie z. B. die Chlorwasserstoffsäure und die Bromwasserstoffsäure, ferner Phosphorsäure, Salpetersäure und Schwefelsäure.

Die Metallsalzkomplexe der Verbindungen der Formel (I) können in einfacher Weise nach üblichen Verfahren erhalten werden, so z. B. durch Lösen des Metallsalzes in Alkohol, z. B. Ethanol, und Hinzufügen zur Verbindung der Formel (I). Man kann Metallsalzkomplexe in bekannter Weise z. B. durch Abfiltrieren, isoliert und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chrytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so z. B. gegen den Erreger des Gurkenmehltaus (Erysiphe cichoracearum), des Apfelmehltaus (Podosphaera leucotricha) oder des Getreidemehltaus (Erysiphe graminis) sowie zur Bekämpfung von Puccinia-Arten, so z. B. gegen den Erreger des Getreiderosts (Puccinia recondita).

In entsprechenden Aufwandkonzentrationen zeigen die erfindungsgemäßen Stoffe auch eine herbizide bzw. wachtstumsregulierende Wirkung.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetiche Granulate aus anorganischen und organischen Mehlen sowie Granulate aus organischem Material wie Sägemehl, Kokosnußschalen, Maiskolben und

Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

In den nachfolgenden Beispielen werden die nachstehend angegebenen Verbindungen als Vergleichssubstanzen eingesetzt:

(A) = 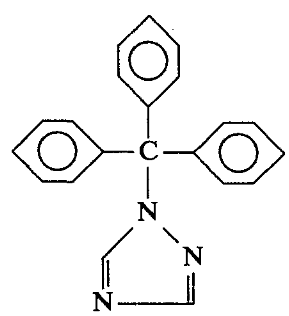

(B) = 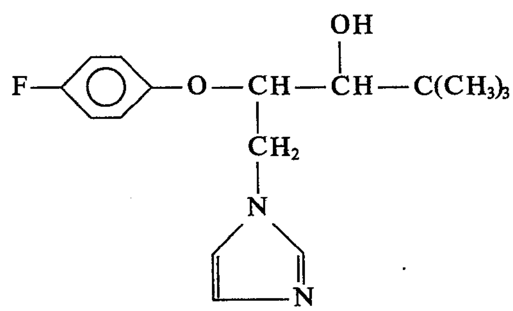

(C) = 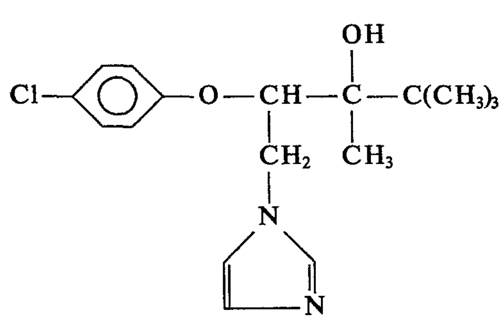

(D) = 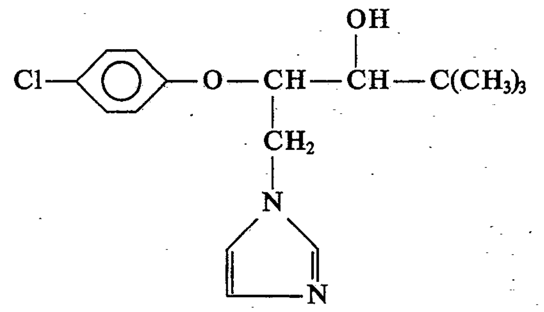

(E) = 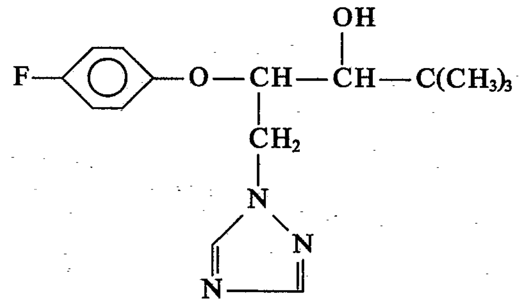

Beispiel A

Sproßbehandlungs-Test/Getreidemehltau/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var. hordei.

Nach 6 Tagen Verweilzeit der Pflanzen bei einer Temperatur von 21 – 22° C und einer Luftfeuchtigkeit von 80 – 90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z. B. die folgenden Verbindungen eine sehr gute Wirkung, die derjenigen der aus dem Stand der Technik bekannten Verbindung (A) überlegen ist:

Verbindungen gemäß Herstellungsbeispielen 17, 18, 19, 20, 21, 22 und 23.

Beispiel B

Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator Alkylaryl-polyglykolether auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100%igen Luftfeuchtigkeit in ein Gewächshaus.

Nach 10 Tagen Verweilzeit der Pflanzen bei einer Temperatur von 20°C und einer Luftfeuchtigkeit von 80 – 90% wertet man den Besatz der Pflanzen mit Rostpusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Rostbefall ist.

In diesem Test zeigen z. B. die folgenden Verbindungen eine sehr gute Wirkung, die derjenigen der aus dem Stand der Technik bekannten Verbindung (B) überlegen ist:

Verbindungen gemäß Herstellungsbeispielen 5, 6, 1, 9, 12 und 13.

## Beispiel C

### Podosphaera-Test (Apfel)/protektiv

| | |
|---|---|
| Lösungsmittel: | 4,7 Gewichtsteile Aceton |
| Emulgator: | 0,3 Gewichtsteile Alkylarylpolyglykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen Luftfeuchtigkeit von 70% im Gewächshaus. Anschließend werden sie durch Bestäuben mit Konidien des Apfelmehltauerregers (Podosphaera leucotricha) inokuliert und in ein Gewächshaus mit einer Temperatur von 21 bis 23° C und einer relativen Luftfeuchtigkeit von 70% gebraucht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

In diesem Test zeigen z. B. folgende Verbindungen eine sehr gute Wirkung, die derjenigen der aus dem Stand der Technik bekannten Verbindungen (C) und (D) überlegen ist:

Verbindungen gemäß Herstellungsbeispielen 2, 6, 7 und 20.

## Beispiel D

### Erysiphe-Test (Gurken)/protektiv

| | |
|---|---|
| Lösungsmittel: | 4,7 Gewichtsteile Aceton |
| Emulgator: | 0,3 Gewichtsteile Alkyl-aryl-polyglykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoreacearum bestäubt. Die Pflanzen werden anschließend bei 23 bis 24° C und bei einer relativen Luftfeuchtigkeit von ca. 75% im Gewächshaus aufgestellt.

Nach 12 Tagen wird der Befall der Gurkenpflanzen bestimmt. Die erhaltenen Boniturwerte werden in Prozent Befall umgerechnet. 0% bedeutet keinen Befall, 100% bedeutet, daß die Pflanzen vollständig befallen sind.

Bei diesem Test zeigen z. B. die folgenden Verbindungen eine sehr gute Wirkung, die derjenigen der aus dem Stand der Technik bekannten Verbindungen (C) und (D) überlegen ist:

Verbindungen gemäß Herstellungsbeispielen 5, 6 und 9.

## Beispiel E

### Myzelwachstums-Test

Verwendeter Nährboden:

| | |
|---|---|
| 20 Gewichtsteile | Agar-Agar |
| 200 Gewichtsteile | Kartoffeldekokt |
| 5 Gewichtsteile | Malz |
| 15 Gewichtsteile | Dextrose |
| 5 Gewichtsteile | Pepton |
| 2 Gewichtsteile | Dinatriumhydrogenphosphat |
| 0,3 Gewichtsteile | Calciumnitrat |

Verhältnis von Lösungsmittelgemisch zum Nährboden:

2 Gewichtsteile Lösungsmittelgemisch
100 Gewichtsteile Agarnährboden

Zusammensetzung Lösungsmittelgemisch:

0,19 Gewichtsteile Dimethylformamid oder Aceton
0,01 Gewichtsteile Emulgator Alkylarylpolyglykolether
1,80 Gewichtsteile Wasser
2 Gewichtsteile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42° C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21° C inkubiert.

Die Auswertung erfolgt je nach Wachstumsgeschwindigkeit der Pilze nach 4 – 10 Tagen. Bei der Auswertung wird das radiale Myzelwachstum auf den behandelten Nährböden mit dem Wachstum auf dem Kontrollnährboden verglichen. Die Bonitierung des Pilzwachstums geschieht mit folgenden Kennzahlen:

1 kein Pilzwachstum,
bis 3 sehr starke Hemmung des Wachstums,
bis 5 mittelstarke Hemmung des Wachstums,
bis 7 schwache Hemmung des Wachstums,
9 Wachtstum gleich der unbehandelten Kontrolle.

Bei diesem Test zeigen z. B. die folgenden Verbindungen eine sehr gute Wirkung, die derjenigen der aus dem Stand der Technik bekannten Verbindungen (B) und (E) überlegen ist:
Verbindungen gemäß Herstellungsbeispielen 9, 13 und 12.

In diesem Versuch wurden die folgenden Pilzarten verwendet:

| | | |
|---|---|---|
| Fusarium culmorum | Pythium ultimum | Phialophora cinerescens |
| Sclerotinia sclerotiorum | Cochliobolus miyabeanus | Helminthosporium gramineum |
| Fusarium nivale | Botrytis cinerea | Mycosphaerella musicola |
| Colletotrichum coffeanum | Verticillium alboatrum | Phytophthora cactorum |
| Rhizotonia solani | Pyricularia oryzae | Pellicularia sasakii |

## Herstellungsbeispiele

### Beispiel 1

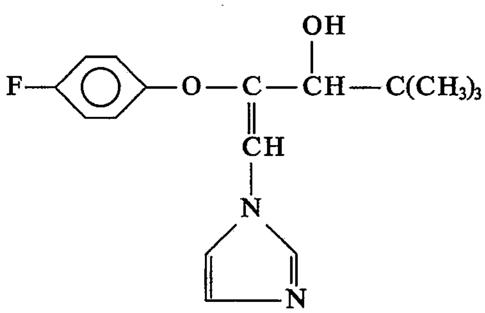

In eine Lösung von 22 g (0,076 Mol) 1-(Imidazol-1-yl)-2-(4-fluorphenoxy)-4,4-dimethyl-1-penten-3-on in 200 ml Methanol werden unter leichter Außenkühlung bei 20 bis 30° C portionsweise 2,9 g (0,076 Mol) Natriumboranat innerhalb von 20 Minuten unter Rühren eingetragen. Nach 3 Stunden wird das Reaktionsgemisch mit 10%iger Essigsäure auf pH 5 gestellt und anschließend unter vermindertem Druck eingedampft.

Der verbleibende ölige Rückstand wird in 250 ml Essigester aufgenommen und zweimal mit je 50 ml Wasser ausgeschüttelt. Nach dem Trocknen der organischen Phase über wasserfreiem Natriumsulfat

wird die Lösung im Vakuum eingedampft, der verbleibende Rückstand in sehr wenig Ether gelöst und langsam mit Petrolether versetzt. Die ausgeschiedenen farblosen Kristalle werden filtriert, mit wenig Petrolether gewaschen und bei 100°C getrocknet. Man erhält 13,7 g (62% der Theorie) 1-(Imidazol-1-yl)-2-(4-fluorphenoxy)-4,4-dimethyl-1-penten-3-ol vom Schmelzpunkt 112–113°C.

Herstellung des Ausgangsproduktes

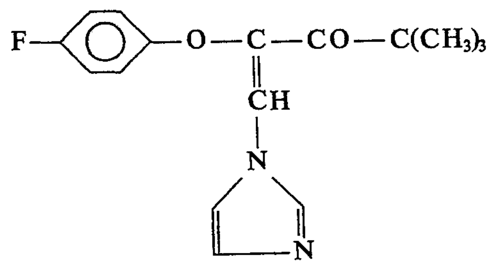

Zu einer Suspension von 90 g (1 Mol) Natrium-imidazol, hergestellt aus Natriummethylat und Imidazol in Methanol, in 2500 ml Acetonitril werden 256,7 g (1 Mol) 1-Chlor-2-(4-fluorphenoxy)-4,4-dimethyl-1-penten-3-on in 150 ml Acetonitril unter Rühren getropft. Danach wird das Reaktionsgemisch 6 Stunden zum Sieden erhitzt. Man läßt auf Raumtemperatur abkühlen und engt durch Abdestillieren des Lösungsmittels im Vakuum ein. Der Rückstand wird mit 1000 ml Essigester aufgenommen, dreimal mit je 200 ml Wasser gewaschen, die organische Phase über Natriumsulfat getrocknet und durch Abdestillieren des Lösungsmittels im Vakuum eingeengt. Man erhält 272,4 g (94,5% der Theorie) rohes 1-(Imidazol-1-yl)-2-(4-fluorphenoxy)-4,4-dimethyl-1-penten-3-on als braunes Öl, das in üblicher Weise über das Nitrat gereinigt wird und dann einen Brechungsindex $n_D^{20}$ = 1,5590 besitzt.

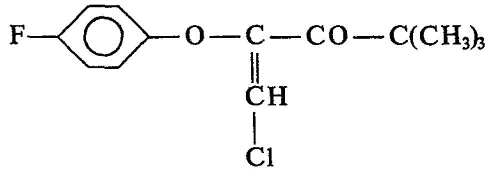

297,5 g (2,5 Mol) Thionylchlorid werden langsam in eine auf 60°C erwärmte Lösung von 404,6 g (1,7 Mol) 1-Hydroxy-2-(4-fluorphenoxy)-4,4-dimethyl-1-penten-3-on in 3000 ml wasserfreiem Toluol eingerührt. Man hält 12 Stunden bei dieser Temperatur und destilliert anschließend das Lösungsmittel und überschüssiges Thionylchlorid ab. Das zurückbleibende Öl wird im Vakuum destilliert. Man erhält 353,3 g (81% der Theorie) 1-Chlor-2-(4-fluorphenoxy)-4,4-dimethyl-1-penten-3-on vom Siedepunkt 95–103°C/0,3 mm.

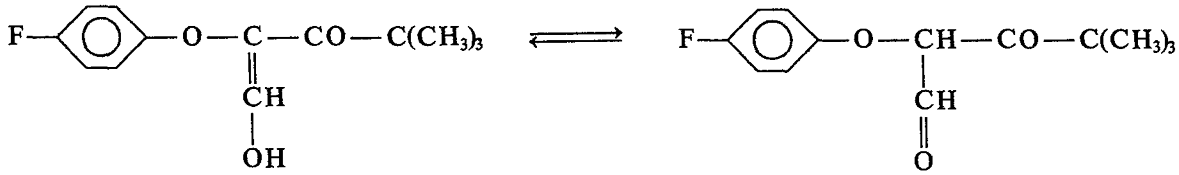

163 g (2,2 Mol) Ameisensäureethylester werden bei 0°C zu einer Lösung von 136 g Natrium-ethylat in 1500 ml Ethanol getropft. Anschließend werden bei 0°C 420 g (2 Mol) 2,2-Dimethyl-4-(4-fluorphenoxy)-butan-3-on langsam eingerührt. Nach 24stündiger Reaktionszeit bei 0°C läßt man auf Raumtemperatur erwärmen und rührt bei dieser Temperatur noch 96 Stunden nach. Das Reaktionsgemisch wird auf 5000 ml Eiswasser gegeben, und die organische Phase wird durch Extrahieren mit Chloroform abgetrennt. Aus dieser Chloroformlösung kann nichtumgesetztes Ausgangsprodukt isoliert und erneut eingesetzt werden. Die wäßrige Phase wird unter Kühlung mit 10%iger Salzsäure angesäuert und das sich ausscheidende Öl in Chloroform aufgenommen. Die Chloroformphase wird über Natriumsulfat getrocknet und durch Abdestillieren des Lösungsmittels im Vakuum eingeengt. Das zurückbleibende Öl wird im Vakuum destilliert. Man erhält 170 g (83% der Theorie, bezogen auf umgesetztes Produkt) 1-Hydroxy-2-(4-fluorphenoxy)-4,4-dimethyl-1-penten-3-on vom Siedepunkt 101–102°C/0,6 mm ($n_D^{20}$ = 1,5132).

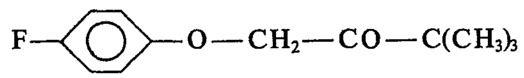

418,3 g (3,11 Mol) 2,2-Dimethyl-4-chlor-butan-3-on werden in eine zum Sieden erhitzte Suspension von 315 g (2,8 Mol) 4-Fluor-phenol und 386,4 g (2,8 Mol) Kaliumcarbonat in 1500 ml Aceton getropft. Man läßt 4 Stunden unter Rückfluß rühren. Nach Abkühlung auf Raumtemperatur wird das ausgeschiedene Salz abfiltriert und das Filtrat im Vakuum eingeengt. Das zurückbleibende Öl wird im Vakuum destilliert. Man erhält 101,5 g (86,2% der Theorie) 2,2-Dimethyl-4-(4-fluorphenoxy)-butan-3-on vom Siedepunkt 83–84° C/0,05 mm ($n_D^{20}$=1,4919).

Beispiel 2

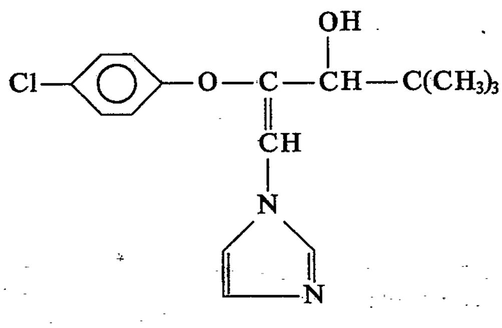

In eine Lösung von 98,8 g (0,33 Mol) 1-(Imidazol-1-yl)-2-(4-chlorphenoxy)-4,4-dimethyl-1-penten-3-on in 1000 ml Methanol werden unter leichter Außenkühlung bei 20 bis 30° C portionsweise 12,4 g (0,33 Mol) Natriumboranat innerhalb von 30 Minuten eingetragen. Nach 3 Stunden wird die Lösung filtriert, das Filtrat mit 10%iger Essigsäure angesäuert und unter vermindertem Druck eingedampft. Der verbleibende feste Rückstand wird in 1,3 l Wasser eingerührt. Das wasserunlösliche Reduktionsprodukt wird abfiltriert und im Vakuum bei 50° C getrocknet. Man erhält 93,7 g (92,55% der Theorie) 1-(Imidazol-1-yl)-2-(4-chlorphenoxy)-4,4-dimethyl-1-penten-3-ol vom Schmelzpunkt 141–143° C in Form farbloser Kristalle.

Herstellung des Ausgangsproduktes

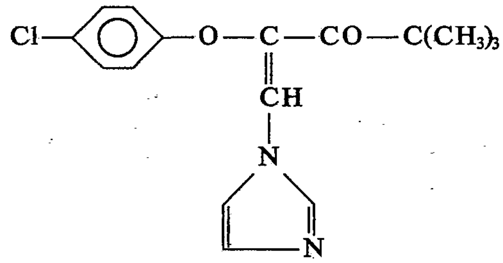

(neue Verfahrensvariante)

13 g (0,05 Mol) 1-Chlor-2-(4-chlorphenoxy)-4,4-dimethyl-1-penten-3-on und 7 g (0,05 Mol) Trimethylsilylimidazol werden in 50 ml Toluol 5 Stunden unter Rückfluß erhitzt. Danach wird das Reaktionsgemisch durch Abdestillieren des Lösungsmittels im Vakuum eingeengt und der ölige Rückstand säulenchromatographisch getrennt (Kieselgel; Essigester zu Chloroform = 1 : 2). Man erhält 9 g (58% der Theorie) 1-(Imidazol-1-yl)-2-(4-chlorphenoxy)-4,4-dimethyl-1-penten-3-on vom Schmelzpunkt 94–95° C.

In entsprechender Weise wie in den Beispielen 1 und 2 angegeben werden die nachfolgenden Verbindungen der allgemeinen Formel:

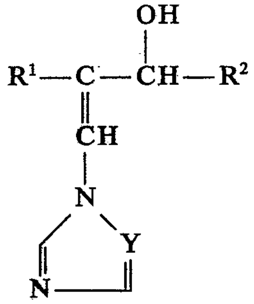

(I)

erhalten:

| Bsp. Nr. | $R^1$ | $R^2$ | Y | Schmelzpunkt (°C) |
|---|---|---|---|---|
| 3 | 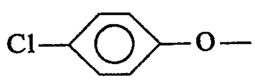 | $C(CH_3)_3$ | CH | 195–97 (xHCl) |
| 4 | 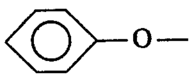 | $C(CH_3)_3$ | CH | 133–34 |
| 5 | 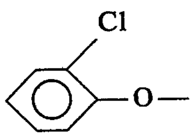 | $C(CH_3)_3$ | CH | 103–05 |
| 6 | 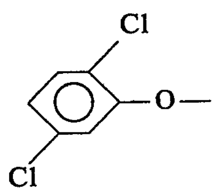 | $C(CH_3)_3$ | CH | 110–22 |
| 7 | 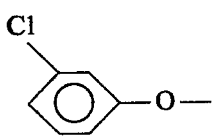 | $C(CH_3)_3$ | CH | 107–08 |
| 8 | 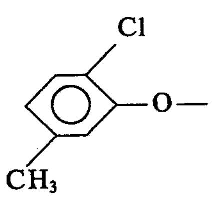 | $C(CH_3)_3$ | CH | 137–38 |
| 9 | 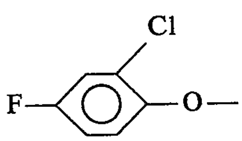 | $C(CH_3)_3$ | CH | 75–88 |
| 10 | 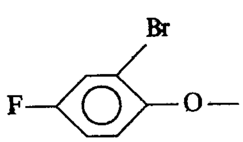 | $C(CH_3)_3$ | CH | 75–93 |
| 11 | 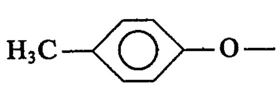 | $C(CH_3)_3$ | CH | 140–41 |
| 12 | 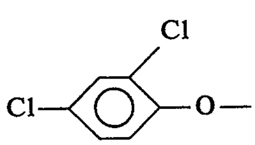 | $C(CH_3)_3$ | CH | 106–08 (Zers.) |
| 13 | 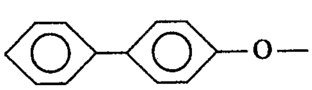 | $C(CH_3)_3$ | CH | 80–82 (Zers.) |
| 14 | 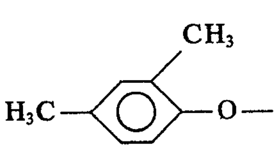 | $C(CH_3)_3$ | CH | 110–14 |

Fortsetzung

| Bsp. Nr. | $R^1$ | $R^2$ | Y | Schmelzpunkt (°C) |
|---|---|---|---|---|
| 15 | 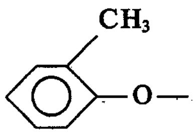 | $C(CH_3)_3$ | CH | 92–95 |
| 16 | 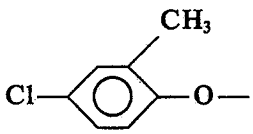 | $C(CH_3)_3$ | CH | 83–85 |
| 17 | 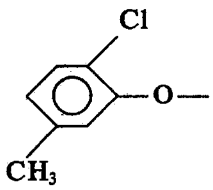 | $C(CH_3)_3$ | N | 150–53 |
| 18 | 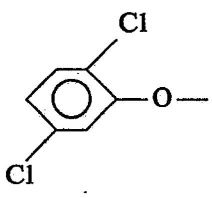 | $C(CH_3)_3$ | N | 136–37 |
| 19 | 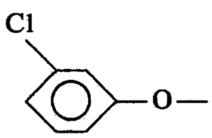 | $C(CH_3)_3$ | N | 98–99 |
| 20 | 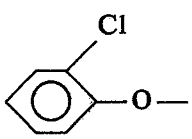 | $C(CH_3)_3$ | N | 75–80 |
| 21 | 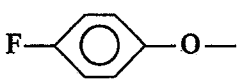 | $C(CH_3)_3$ | N | 118–19 |
| 22 | 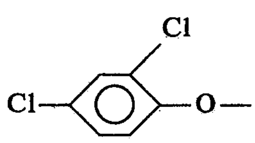 | $C(CH_3)_3$ | N | 125–30 |
| 23 | 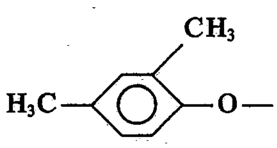 | $C(CH_3)_3$ | N | 118–19 |
| 24 | 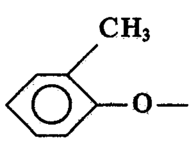 | $C(CH_3)_3$ | N | 73–78 |
| 25 | 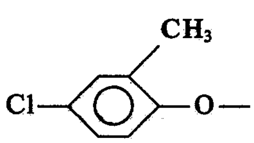 | $C(CH_3)_3$ | N | 117–18 |

Fortsetzung

| Bsp. Nr. | $R^1$ | $R^2$ | Y | Schmelzpunkt (°C) |
|---|---|---|---|---|
| 26 | 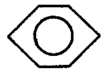 | 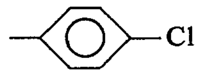 | N | 116–18 |
| 27 | 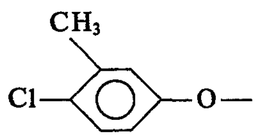 | $C(CH_3)_3$ | CH | 148–49 |
| 28 | 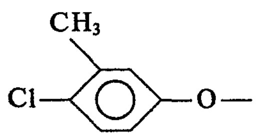 | $C(CH_3)_3$ | N | 108–09 |
| 29 | 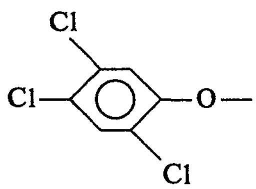 | $C(CH_3)_3$ | CH | 128–30 |

**Patentansprüche**

1. Azolyl-alkenole der allgemeinen Formel

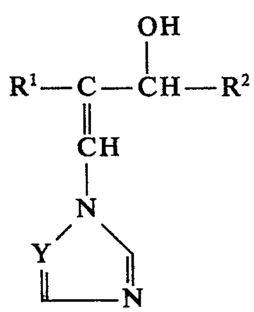

(I)

in welcher

$R^1$ für Phenoxy oder Phenyl steht, wobei die genannten Reste substituiert sein können durch Halogen, Alkyl mit 1 bis 4 Kohlenstoffatomen und durch gegebenenfalls halogensubstituiertes Phenyl,

$R^2$ für Alkyl mit 1 bis 4 Kohlenstoffatomen oder für Phenyl steht, wobei letzteres substituiert sein kann durch Halogen, Alkyl mit 1 bis 4 Kohlenstoffatomen und durch gegebenenfalls halogensubstituiertes Phenyl, und

Y für ein Stickstoffatom oder die CH-Gruppe steht,

und deren physiologisch verträglichen Säureadditions-Salze und Metallsalz-Komplexe.

2. Verfahren zur Herstellung von Azolyl-alkenolen der allgemeinen Formel

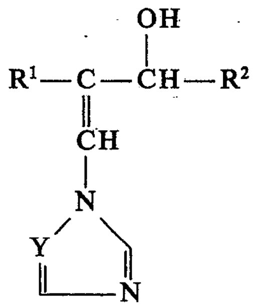

(I)

in welcher

$R^1$ für Phenoxy oder Phenyl steht, wobei die genannten Reste substituiert sein können durch Halogen, Alkyl mit 1 bis 4 Kohlenstoffatomen und durch gegebenenfalls halogensubstituiertes Phenyl,

$R^2$ für Alkyl mit 1 bis 4 Kohlenstoffatomen oder für Phenyl steht, wobei letzteres substituiert sein kann durch Halogen, Alkyl mit 1 bis 4 Kohlenstoffatomen und durch gegebenenfalls halogensubstituiertes Phenyl, und

Y für ein Stickstoffatom oder die CH-Gruppe steht,

dadurch gekennzeichnet, daß man Azolyl-alkenone der allgemeinen Formel

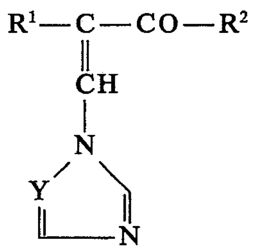

(II)

in welcher
$R^1$, $R^2$ und Y die oben angegebenen Bedeutungen haben,
nach bekannten Methoden in üblicher Weise reduziert und gegebenenfalls an die so erhaltenen Verbindungen der Formel (I) anschließend eine Säure oder ein Metallsalz addiert.

3. Verfahren gemäß Anspruch 2 zur Herstellung von Azolyl-alkenolen der allgemeinen Formel I, dadurch gekennzeichnet, daß man zunächst zur Herstellung der Vorprodukte der Formel II die 1-Halogen-ethen-Derivate der Formel

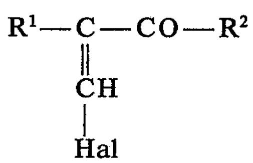

(III)

in welcher

$R^1$ und $R^2$ die in Anspruch 2 angegebenen Bedeutungen haben, und
Hal für Halogen steht,

mit Trimethylsilyl-azolen der Formel

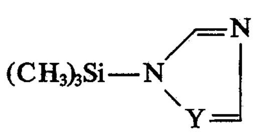

(IV)

in welcher
Y die in Anspruch 2 angegebene Bedeutung hat,
in Gegenwart eines Verdünnungsmittels umsetzt, und das hierbei erhaltene Azolyl-alkenon der Formel II in Anspruch 2 nach bekannten Methoden in üblicher Weise reduziert und gegebenenfalls an die so erhaltenen Verbindungen der Formel I anschließend eine Säure oder ein Metallsalz addiert.

4. Fungizide Mittel für den Pflanzenschutz, gekennzeichnet durch einen Gehalt an mindestens einem Azolyl-alkenol der Formel I in Ansprüchen 1 und 2.

5. Verwendung von Azolyl-alkenolen der Formel I in Ansprüchen 1 und 2 zur Bekämpfung von phytopathogenen Pilzen im Pflanzenschutz.

6. Verfahren zur Herstellung von fungiziden Mitteln für den Pflanzenschutz, dadurch gekennzeichnet, daß man Azolyl-alkenole der Formel I in Ansprüchen 1 und 2 mit Streckmitteln und/oder oberflächenaktiven Mitteln vermischt.

## Claims

1. Azolyl-alkenols of the general formula

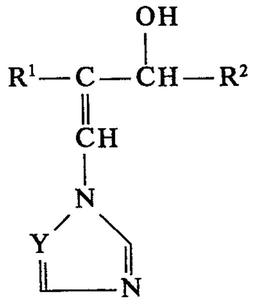

(I)

in which

$R^1$ represents phenoxy or phenyl, it being possible for the radicals mentioned to be substituted by halogen, alkyl with 1 to 4 carbon atoms or by optionally halogen-substituted phenyl,

$R^2$ represents alkyl with 1 to 4 carbon atoms or phenyl, it being possible for the latter to be substituted by halogen, alkyl with 1 to 4 carbon atoms or by optionally halogen-substituted phenyl, and

Y represents a nitrogen atom or the CH group,

and physiologically acceptable acid addition salts and metal salt complexes thereof.

2. Process for the preparation of azolyl-alkenols of the general formula

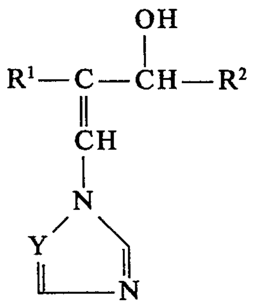

(I)

in which

$R^1$ represents phenoxy or phenyl, it being possible for the radicals mentioned to be substituted by halogen, alkyl with 1 to 4 carbon atoms or by optionally halogen-substituted phenyl,

$R^2$ represents alkyl with 1 to 4 carbon atoms or phenyl, it being possible for the latter to be substituted by halogen, alkyl with 1 to 4 carbon atoms or by optionally halogen-substituted phenyl, and

Y represents a nitrogen atom or the CH group,

characterised in that azolyl-alkenones of the general formula

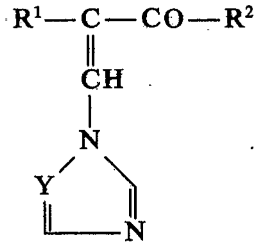

(II)

in which
$R^1$, $R^2$ and Y have the meanings indicated above,
are reduced in the customary manner by known methods and optionally an acid or a metal salt is then added onto the compounds of the formula (I) thus obtained.

3. Process according to Claim 2 for the preparation of azolyl-alkenols of the general formula I, characterised in that first of all, to prepare the starting products of the formula II, the 1-halogeno-ethene-derivatives of the formula

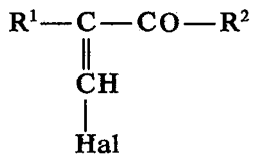

(III)

in which

$R^1$ and $R^2$ have the meanings indicated in Claim 2, and
Hal represents halogen,

are reacted with trimethylsilyl-azoles of the formula

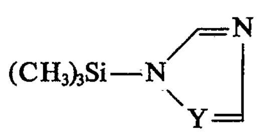

(IV)

in which
Y has the meaning indicated in Claim 2,
in the presence of a diluent and the azolyl-alkenone of the formula II in Claim 2 which is thereby obtained is reduced in the customary manner by known methods and an acid or a metal salt is then optionally added onto the compounds of the formula I thus obtained.

4. Fungicidal agents for plant protection, characterised in that they contain at least one azolyl-alkenol of the formula I in Claims 1 and 2.

5. Use of azolyl-alkenols of the formula I in Claims 1 and 2 for combating phytopathogenic fungi in plant protection.

6. Process for the preparation of fungicidal agents for plant protection, characterised in that azolyl-alkenols of the formula I in Claims 1 and 2 are mixed with extenders and/or surface-active agents.

**Revendications**

1. Azolyl-alcénols de formule générale:

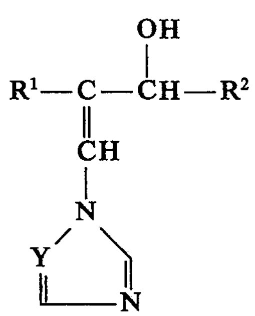

(I)

dans laquelle

$R^1$ représente un reste phénoxy ou phényle, les restes mentionnés pouvant être substitués par un halogène, un radical alkyle ayant 1 à 4 atomes de carbone et par un radical phényle éventuellement substitué par un halogène,
$R^2$ est un reste alkyle ayant 1 à 4 atomes de carbone ou un reste phényle, ce dernier pouvant être substitué par un halogène, un radical alkyle ayant 1 à 4 atomes de carbone et par un reste phényle éventuellement substitué par un halogène, et
Y est un atome d'azote ou le groupe CH,

et leurs sels d'addition d'acides et leurs complexes de sels métalliques acceptables du point de vue physiologique.

2. Procédé de production d'azolyl-alcénols de formule générale:

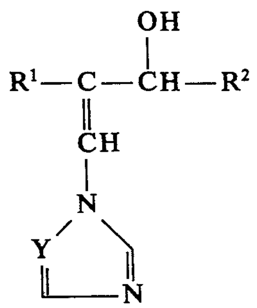

(I)

dans laquelle

$R^1$ représente un reste phénoxy ou phényle, les restes mentionnés pouvant être substitués par un halogène, un radical alkyle ayant 1 à 4 atomes de carbone et par un radical phényle éventuellement substitué par un halogène,
$R^2$ est un reste alkyle ayant 1 à 4 atomes de carbone ou un reste phényle, ce dernier pouvant être substitué par un halogène, un radical alkyle ayant 1 à 4 atomes de carbone et par un reste phényle éventuellement substitué par un halogène, et
Y est un atome d'azote ou le groupe CH,

caractérisé en ce qu'on réduit d'une manière classique des azolyl-alcénones de formule générale:

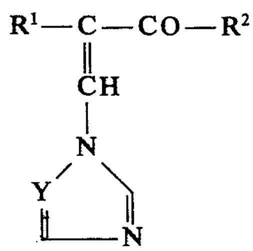

(II)

dans laquelle
$R^1$, $R^2$ et Y ont les définitions indiquées ci-dessus,
par des opérations connues et, le cas échéant, on additionne ensuite un acide ou un sel métallique sur les composés ainsi obtenus de formule (I).

3. Procédé suivant la revendication 2 pour la production d'azolyl-alcénols de formule générale I, caractérisé en ce qu'on fait tout d'abord réagir, pour la production des composés de départ de formule II, les dérivés de 1-halogéno-éthènes de formule:

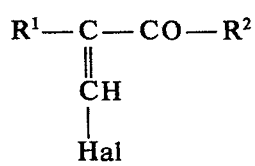

(III)

dans laquelle

$R^1$ et $R^2$ ont les définitions indiquées dans la revendication 2, et
Hal représente un halogène,

avec des triméthylsilyl-azoles de formule:

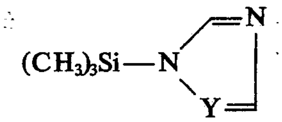

(IV)

dans laquelle

Y a la définition indiquée dans la revendication 2,

en présence d'un diluant, et on réduit l'azolyl-alcénone ainsi obtenue de formule II suivant la revendication 2 d'une manière par des opérations connues et, le cas échéant, on additionne ensuite un acide ou un sel métallique sur les composés de formule I ainsi obtenus.

4. Compositions fongicides pour la protection des plantes, caractérisées par une teneur en au moins un azolyl-alcénol de formule I suivant les revendications 1 et 2.

5. Utilisation d'azolyl-alcénols de formule I suivant les revendications 1 et 2 pour la lutte contre des champignons phytopathogènes dans la protection des plantes.

6. Procédé de production de compositions fongicides pour la protection des plantes, caractérisé en ce qu'on mélange des azolyl-alcénols de formule I suivant les revendications 1 et 2 avec des diluants et/ou des agents tensio-actifs.